(19) Europäisches Patentamt
European Patent Office
Office européen des brevets

(11) **EP 2 698 182 A1**

(12) **EUROPEAN PATENT APPLICATION**

(43) Date of publication:
**19.02.2014 Bulletin 2014/08**

(51) Int Cl.:
***A61M 5/44*** *(2006.01)*

(21) Application number: **12180279.7**

(22) Date of filing: **13.08.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **seiratherm GmbH**
**91074 Herzogenaurach (DE)**

(72) Inventors:
• **Reichthalhammer Dr. Thomas**
**85716 Unterschleißheim (DE)**
• **Roth , Dr. Matthias**
**85376 Giggernhausen (DE)**

(74) Representative: **Vossius & Partner**
**Siebertstrasse 4**
**81675 München (DE)**

(54) **Method and device for adjusting the temperature of medical liquids**

(57) The present invention relates to a Method and a device for adjusting the temperature of medical liquids, comprising: Providing an incoming volumetric flow (Fi) from a fluid supply (1); Seperating the incoming volumetric flow (Fi) into two partial volumetric flows (F1, F2); Adjusting the fluid temperature (T1, T2) of each of the partial volumetric flows (F1, F2) to substantially constant target temperatures (Ttl, Tt2) of the partial volumetric flows; and volumetric flow controlled merging of the partial volumetric flows (F1, F2) to an output volumetric flow (Fo).

Fig. 1

**Description**

The invention relates to a method and a device for adjusting the temperature of medical liquids. Such method and device may, for instance, be used for adjusting the temperature of a medical liquid used for manipulating the body temperature of a patient. For instance, the temperature of a patient may be adjusted in order to benefit from the positive effects of hypothermia.

Document WO 2009/056640 discloses an apparatus and method for adjusting or stabilizing the body temperature of a patient. The apparatus comprises at least one temperature sensor for measuring a temperature of an infusion fluid and a control unit comprising input channels for reading in sensor readings from said temperature sensor and for reading in measurement data representing the patient's body temperature. Furthermore, the apparatus comprises means for controlling, depending on said measurement data, volume flow of infusion fluid. The apparatus is, in one embodiment, connected to two fluid supply containers containing infusion fluid, wherein the containers have different temperatures. These infusion fluids are in fluid connection with a y-shaped connector of a flexible tube. The flow rate of the two infusion fluids from the containers of different temperature are controlled by controller.

Hence, the apparatus according to WO 2009/056640 suggests two separate, pre-cooled or prewarmed fluid containers and a subsequent feedback-controlled mixture to target temperature. Before the fluids could be infused to the patient, the medical personnel would be required to charge the device with infusions fluids, start the device in order to achieve the pre-cooling or pre-warming within the separate supply containers and only once target temperatures are achieved - it would be possible to start the infusion therapy.

It is an object of the present invention to overcome or ameliorate the aforementioned drawbacks of the prior art and to provide an improved and/or alternative and/or additional method or device for controlling a temperature of a patient.

It is one preferred object of the present invention to provide a device with a simple and robust design in order to further improve the failsafe operation. Moreover, it is a preferred object of the present invention to provide a cost efficient design, preferably with a reduced number of parts and preferably using cheap and standardized components which may also further reduce the assembly costs. Another preferred object of the present invention is to provide a device having a compact design which may not limit the access to a patient during operation. It is also a preferred object of the present invention to provide a device with an improved operability and/or an increased ease of use during operation and set up of the device. Preferably, only minor changes to the routine of the personnel are necessary. Also, it is one preferred object of the present invention to avoid long setup times.

The object(s) underlying the present invention is (are) particularly solved by the features defined in the independent claims. The dependent claims relate to preferred embodiments of the present invention. Further additional and/or alternative aspects are discussed below.

Thus, at least one of the preferred objects of the present invention is solved by a method for adjusting the temperature of medical liquid(s). Preferably, the method comprises the step of providing at least one, preferably one, incoming volumetric flow Fi or a flow rate from at least one, preferably one, fluid supply. Moreover, the method preferably comprises the step of separating the incoming volumetric flow Fi into at least two, preferably two, partial volumetric flows F1, F2, and the step of adjusting or changing the fluid temperature T1, T2 of each of the partial volumetric flows F1, F2 to substantially constant target temperatures Tt1, Tt2, preferably to constant target temperatures Tt1, Tt2, of the partial volumetric flows, and preferably the step of volumetric flow control merging of the partial volumetric flow F1, F2 to an output volumetric flow Fo.

A medical liquid could be understood as any kind of fluid used in the medical environment. For instance, the medical liquid could be blood, particularly extra corporal fluids like blood, dialysis liquids or substitute liquids, more preferably an infusion liquid such as electrolyte solutions such as NaCl and/or Ringer solutions and/or Jonosteril®.

The term "constant target temperatures Tt1, Tt2" is to be understood as the temperatures of the partial volumetric flows that are constant, preferably within the operating range of the device, namely within the operating range of the heat exchanger. With other words, the constant target temperatures are defined or known temperatures of the fluid of the respective partial volumetric flows before merging or mixing. Preferably, the constant target temperatures are the temperatures of the partial volumetric flows F1, F2 at the exit of the heat exchanger. The term "substantially" of the expression "substantially constant target temperatures" means that the constant target temperature value may comprise small deviations within a tolerance range which are not of importance or negligible for the overall adjustment of the temperature of medical liquids, namely for the adjustment of the output volumetric flow Fo and, preferably, its temperature. For example, the tolerance range of the constant target temperatures Tt1, Tt2 may be of the range +/- 2°C or +/-1°C of the constant target temperatures. The term fluid supply relates to any kind of reservoir or supply being adapted to carry the medical liquid. For instance, the fluid supply might be one or several infusion bags, most preferably only one infusion bag is used as a fluid supply.

The term "volumetric flow controlled merging" relates to the mixing or merging or unification of at least two partial volumetric flows to an output volumetric flow Fo wherein the mixing or merging ratio between the at least two partial volumetric flows is controlled by controlling the volumetric flow of at least one, preferably both or all, of the partial volumetric flows. The the mixing or merging ratio is preferably adjusted depending on the desired output temperature To of the output volumetric flow Fo. Since the temperature of the at least two partial volumetric flows F1, F2 are the known substantial constant target temperatures Tt1, Tt2, the output temperature To can be calculated using the formula:

$$(1) \qquad To = (Tt1*F1+Tt2*F2)/Fo,$$

wherein
Fo is the output volumetric flow,
To is the temperature of the adjusted medical liquid or with other words, the output temperature To of the output volumetric flow Fo,
Tt1 is the first substantially constant target temperature,
Tt2 is the second substantially constant target temperature,
F1 first partial volumetric flow, and
F2 second partial volumetric flow.

It is noted that the substantial constant target temperatures Tt1, Tt2 of the at least two partial volumetric flows preferably have constant but different temperatures, particularly in order to be able to adjust the temperature To of the medical liquid. Moreover, the temperature of the medical liquid can only be adjusted to a temperature between the highest constant target temperature and the lowest constant target temperature. Preferably, the device is connected to one fluid supply containing one medical liquid. The medical liquid is provided to the device and then preferably separated by a separator to two partial flows. Alternatively, two incoming volumetric flows of the same or different incoming temperature Ti may be supplied to the device.

Accordingly, the set-up of the apparatus may involve a reduced number of control parameters for the control algorithm as the mixture of the infusion may solely be based on a volume-mixture of the two partial flows, each having a constant temperature after the heat exchange within the electro-thermal element. The apparatus preferably does not require the medical personnel to utilize fluids out of refrigerator or warming oven. The apparatus preferably does not require any similar prior art refrigerator or warming oven within the apparatus itself. The apparatus preferably does not require pre-cooling or pre-warming of the fluids before the infusion therapy could be started. The device according to the present invention is preferably designed to utilize fluids at room temperature, thus representing the usual clinical routine. Medical fluids e.g. NaCl infusions are normally stored with room-temperature and usually infused via an intravenous access via gravitation or with a infusion pump without any pre-cooling or pre-warming. It is preferably possible for the medical personnel to use the fluids at room temperature. The personnel may immediately start with the infusion therapy without any delay caused by the setup. Hence, the application of the device would generally represent the usual clinical routine. It is thus possible to adjust the temperature of a medical liquid with a simple method using a device with a simple and robust design and operability.

The device is preferably designed to use only one electro-thermal heat exchanger (e.g. one Peltier-Element using the cold and warm end of the heat exchanger), thus reducing the number of required parts / heat exchangers for the device itself. Moreover, no temperature sensors may be needed, particularly for each partial flow. In addition, even a sensorless or at least a device without temperature-sensors may thus be implemented. The parts used for the device may predominantly be standard components which may reduce the overall production costs.

Preferably, the system may not need a temperature adjusting means for the fluid supply such as a cooler. Moreover, the device may only be connected to one fluid supply instead of two fluid supplies. Accordingly, the overall dimensions of the system and/or the device may be reduced so that a compact design is achieved.

Preferably, only one tube needs to be connected between the device and the fluid supply. Similarly only one tube needs to be connected to the outlet of the device. Accordingly, the steps to be carried out for the initial setup are reduced compared to prior art devices having two separate fluid supplies, particularly pre-heated and/or pre-cooled fluid supplies, connected to the device.

In one preferred embodiment the incoming fluid temperature T1, T2 of the partial volumetric flows F1, F2 is adjusted with a heat exchanger. The heat exchanger is adapted or adjusted or designed to provide the substantially constant target temperatures Tt1, Tt2 of the two partial volumetric flows up to a respective maximum partial volumetric flow Fm1, Fm2. The method may comprise a heat exchanger with at least one heating element and/or at least one cooling element.

The device is preferably designed to operate safely and provide constant target temperatures within the operating range of the device. The operating parameters of the device and/or the heat exchanger may be defined by:

a) the maximum volumetric flow supplied to the user, which is preferably approximately 5 litres per hour or, preferably, 10 litres per hour within a short term;

b) the maximum substantial constant target temperatures Ttx, which may be, for instance:

aa) a substantial constant target fluid temperature (Tt1) of a partial volumetric flow (F1) of preferably about 42°C or 45°C as an upper limit, for instance obtained by a heating element, and/or

bb) a substantial constant target fluid temperature (Tt2) of a partial volumetric flow (F2) of preferably about 4°C as a lower limit, for instance obtained by a cooling element;

c) the incoming fluid temperatures of the incoming volumetric flow or fluid Ti which may preferably be within the range of 6°C and 42°C.

Normally the incoming fluid temperature is similar to the room temperature, e.g. in the range from 19°C to 22°C. Starting from this temperature, the device may be operated to provide an output flow to the user of a target temperature (=fluid output temperature to the user) of between about 4°C and 45°C. This may be assumed to be the standard operating condition. However, a worst case scenario for dimensioning the heat exchanger would be providing a first partial volumetric flow (upper limit) with a volumetric flow to the user of 5 litres per hour, or 10 litres in shot term, at a substantial constant target temperature (=fluid output temperature to the user) of about 45°C wherein the heat exchanger is supplied with an incoming fluid having a fluid temperature of 6°C. Vice versa, a second worst case scenario for dimensioning the heat exchanger would be providing a second partial volumetric flow (lower limit) with a volumetric flow to the user of 5 litres per hour, or 10 litres in short term, at a substantial constant target temperature (= fluid output temperature to the user) of about 4°C wherein the heat exchanger is supplied with an incoming fluid with a temperature of 42 °C.

Preferably, the fluid supply and/or the partial volumetric flows F1, F2 substantially has/have the same or substantially the same incoming temperature Ti, Ti1, Ti2. With other words, it is, though preferred, not only possible to supply a fluid with one incoming fluid temperature but also with two or several income fluid temperatures which may vary within above-mentioned range of incoming fluid temperatures (6°C to 42°C).

Preferably, at least the electrical power input of the heat exchanger is controlled based on the at least two, preferably two, partial volumetric flows F1, F2. Additionally or alternatively at least the electrical power input of the heat exchanger is controlled based on the output volumetric flow Fo.

With the known (substantially) constant target temperatures Tt1, Tt2 and the known at least two, preferably two, partial volumetric flows F1, F2 and/or known output volumetric flow Fo, either measured by a flow meter or calculated or derived by the information of the fluid pumps, for instance their power consumption, the respective requested amount of heating energy and/or amount of cooling may be calculated. Since the relationship between the electrical power input to the heat exchanger and the respective amount of heating energy and/or amount of cooling energy supplied by the heating element and/or the cooling element to the respective partial volumetric flows (F1, F2) is known, the requested electrical power input may be calculated by a controller.

Preferably, the heat exchanger comprises at least one, preferably one, thermoelectric heat exchanger, preferably a Peltier element. The Peltier element may be used for cooling and/or heating. Preferably, the Peltier element is used for cooling and heating. With other words, the Peltier element comprises or constitutes the heating element and the cooling element. Preferably, a Peltier element may provide about 120W cooling energy using a water quench. Such Peltier element, generally has a degree of efficiency of about 40%. Therefore, it may consume around 300W. The device may comprise one Peltier element, more preferably two or even more Peltier elements may be used. Preferably, one Peltier element is used for each partial volumetric flow. Also a combination of one Peltier element adjusting the second constant target fluid temperature (Tt2) of the second partial volumetric flow (cooling flow) and an electrical heating element such as a resistance heater for heating the first constant target fluid temperature (Tt1) of the first partial volumetric flow (heating flow) is preferred.

Preferably, the heat exchanger comprises a buffer or two or more buffers. The buffers may be associated or thermally coupled with the partial volumetric flows F1, F2 and may be adapted for heating and/or cooling the partial volumetric flows. Preferably, the one or more buffers are thermally isolated from each other. The at least one buffer may comprise an aluminum block and/or a water quench or water reservoir. Preferably, the at least one buffer is adapted to dampen the control response of the heat exchanger. Preferably, the at least one buffer reduces or eliminates overshooting and/or undershooting. Preferably, the at least one buffer is adapted to offset or reduce the thermal inertia of the heat exchanger. To offset or to reduce the thermal inertia of the heat exchanger preferably means that the at least one buffer is adapted to provide the substantially constant target temperatures (Tt1, Tt2) of the partial volumetric flows up to the maximum partial volumetric flows (Fm1, Fm2), particularly when a delayed control response of the heat exchanger needs to be compensated.

The device for adjusting the temperature of medical liquid(s) preferably comprises a heat exchanger adapted to adjust or change the fluid temperature of each of the partial volumetric flows to substantially constant target temperatures Tt1, Tt2, preferably to constant target temperatures Tt1, Tt2, of the partial volumetric flows. It furthermore preferably comprises at least two, preferably two, volumetric flow actuators controlling the partial volumetric flows and at least one, preferably one, mixing or merging element, such as a Y-connector or chamber, merging the partial volumetric flows F1, F2 to an output volumetric flow Fo. Preferably, the device comprises a separator being connectable to at least one, preferably one, fluid supply separating (an) incoming volumetric flow(s) Fi into at least two, preferably two, partial volumetric flows F1, F2.

The heat exchanger is preferably adapted, adjusted and/or designed to provide (substantially) constant target temperatures Tt1, Tt2 of the partial volumetric flows, preferably up to the maximum partial volumetric flows Fm1, Fm2. Preferably, the heat exchanger comprises at least one heating element and/or at least one cooling element. Preferably, the at least the electrical power input of the heat exchanger is controlled based on the at least two, preferably two, partial volumetric flows and/or on the output of the volumetric flow. Preferably, the device, more preferably the heat exchanger, comprises at least one, preferably one, thermoelectric heat exchanger or Peltier element. Preferably, the heat exchanger comprises at least one buffer being associated or thermally coupled with the partial volumetric flows and preferably adapted for heating and/or cooling the partial volumetric flows. Moreover, the at least one buffer may be adapted to dampen the temperature variance within itself e.g. in case of spontaneous volumetric need. Preferably, the at least one buffer is adapted to reduce or offset the thermal inertia of the heat exchanger. Preferably, the two volumetric flow actuators may be pumps, preferably speed-controlled pumps, and most preferably peristaltic pumps. Moreover, a preferred system may comprise the device and at least one fluid supply.

Alternatively or additionally, the application relates to the following aspects:

1. Method for adjusting the temperature of medical liquid(s), comprising:

Providing at least one, preferably one, incoming volumetric flow or flow rate Fi from at least one, preferably one, fluid supply 1;

Preferably, seperating the incoming volumetric flow Fi into at least two, preferably two, partial volumetric flows F1, F2;

Adjusting or changing the fluid temperature T1, T2 of each of the partial volumetric flows F1, F2 to substantially constant target temperatures Tt1, Tt2 of the partial volumetric flows;

volumetric flow controlled merging of the partial volumetric flows F1, F2 to an output volumetric flow Fo.

2. The method of aspect 1, wherein the fluid temperature T1,T2 of the partial volumetric flows F1, F2 is adjusted with a heat exchanger 2, the heat exchanger 2 being adapted or adjusted or designed to provide the substantially constant target temperatures Tt1, Tt2 of the partial volumetric flows preferably up to a respective maximum partial volumetric flow Fm1, Fm2.

3. The method of any one of the preceding aspects, wherein the heat exchanger 2 comprises at least one heating element 3 and/or at least one cooling element 4.

4. The method of any one of the preceding aspects, wherein the fluid supply 1 and/or the partial volumetric flows F1, F2 substantially has/have the same or substantially the same incoming fluid temperature Ti, Ti1, Ti2.

5. The method of any one of the preceding aspects, wherein at least the electrical power input of the heat exchanger 2 is controlled based on the partial volumetric flows F1, F2 and/or on the output volumetric flow Fo.

6. The method of any one of the preceding aspects, wherein the heat exchanger 2 comprises at least one, preferably one, thermoelectric heat exchanger, preferably a Peltier element.

7. The method of any one of the preceding aspects, wherein the heat exchanger 2 comprises a buffer or two or more buffers 5, 6, the buffer(s) 5, 6 being associated or thermally coupled with the partial volumetric flows F1, F2 and adapted for heating and/or cooling the partial volumetric flows F1, F2.

8. The method of aspect 7, wherein the buffer(s) 5, 6 is/are adapted to dampen the control response of the heat exchanger 2, and/or
are adapted to offset or reduce the thermal inertia of the heat exchanger 2.

9. Device for adjusting the temperature of medical liquid(s), comprising:

Preferably, a separator 7 being connectable to at least one, preferably one fluid supply 1 separating at least one, preferably one, incoming volumetric flow or flow rate Fi into at least two, preferably two, partial volumetric flows F1, F2;

a heat exchanger 2 adapted to adjust or change the fluid temperature T1, T2 of each of the partial volumetric flows F1, F2 to substantially constant target temperatures Tt1, Tt2 of the partial volumetric flows;

at least two, preferably two, volumetric flow actuators 8, 9 controlling the partial volumetric flows F1, F2; and

at least one, preferably one, merging element 10 or Y- connector or , chamber merging the partial volumetric flows F1, F2 to an output volumetric flow Fo.

10. The device of aspect 9, wherein the heat exchanger is adapted or adjusted or designed to provide the substantially constant target temperatures Tt1, Tt2 of the partial volumetric flows preferably up to the maximum partial volumetric flows Fm1, Fm2.

11. The device of aspects 9 or 10, wherein the heat exchanger comprises at least one heating element 3 and/or at least one cooling element 4.

12. The device of any one of the preceding aspects 9 to 11, wherein at least the electrical power input of the heat exchanger 2 is controlled based on the partial volumetric flows F1, F2 and/or on the output volumetric flow FO; and/or wherein the heat exchanger 2 comprises at least one, preferably one, thermoelectric heat exchanger, preferably a Peltier element.

13. The device of any one of the preceding aspects 9 to 12, wherein the heat exchanger comprises a buffer or two or more buffers 5, 6 being associated or thermally coupled with the partial volumetric flows F1, F2 and adapted for heating and/or cooling the partial volumetric flows F1, F2.

14. The device of aspect 13, wherein the buffer(s) 5, 6 is/are adapted to dampen the control response of the heat exchanger 2, and/or
are adapted to offset or reduce the thermal inertia of the heat exchanger 2.

15. The device of any one of the preceding aspects 9 to 14, wherein the two volumetric flow actuators are speed controlled pumps 8, 9, preferably peristaltic pumps 8, 9.

16. System, comprising the device of any one of the preceding aspects 9 to 14 and at least one, preferably one, fluid supply 1.

The present invention will become more fully understood from the detailed description given hereinafter and the accompanying drawings which are given by way of illustration only, and thus, are not limitative of the present invention, and wherein:

FIG. 1 is a schematic view of the device in one preferred embodiment, and
FIG. 2 is a schematic view of another preferred embodiment.

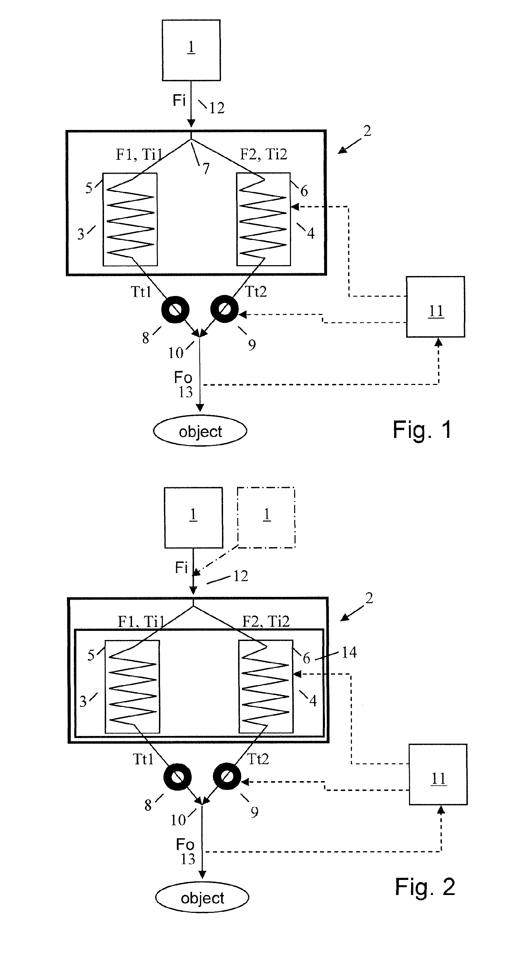

The device 2 of Fig. 1 is connected to one fluid supply 1 via a fluid connection, preferably a tube, 12. The incoming fluid flow Fi is divided in separator 7 into two partial volumetric flows F1 and F2 having respective incoming fluid temperatures Ti1 and Ti2. Preferably, particularly when origin from one fluid supply, Ti1 is equal to Ti2. The separated two partial volumetric flows are then directed to heating element 3 and cooling element 4, respectively. Heating element 3 and cooling element 4 are integrated into heat exchanger 2. Both partial volumetric flows flow through fluid pathways of heating element 3 and cooling element 4. Such pathways may have, e.g., a zigzag or meander shape. While flowing through the fluid pathway, the fluid temperature of each partial volumetric flow F1, F2, if Ti1 and/or Ti2 differ from the respective temperature provided by the heat exchanger, are adjusted. Moreover, heat exchanger 2 of Fig. 1 comprises buffer elements 5, 6, which are thermally connected to heating element 3 and cooling element 4 and also with the respective partial volumetric flow. Heating element 3 and cooling element 4 are preferably thermally isolated from each other. Similarly, also buffer 5 and buffer 6 are preferably thermally isolated from each other. At the exit of the heating element 3 and cooling element 4, the two partial volumetric flows F1 and F2 have a fluid temperature T1, T2 which preferably substantially correspond to the (substantial) constant target temperatures Tt1 and Tt2, respectively. The partial volumetric flows or flow rates F1, F2 are preferably controlled by a controller 11 being in communication with two volumetric flow actuators 8, 9, here embodied as peristaltic pumps 8, 9. Controller 11 may not only send a control signal to peristaltic pumps 8, 9 but may also receive a control feedback of the respective pump 8, 9, preferably relating to speed or power consumption. The partial volumetric flows F1, F2 are merged or mixed in merging element 10, which is here configured as a Y-connector 10. The partial volumetric flow Fo is channelled through a tube 13 to an object, for instance a supply storage, further flow adaptation means or a patient. In a preferred embodiment the device may additionally comprise sensors in communication with controller 11, adapted to measure the flow parameter(s), for instance the temperature, flow rate and/or pressure of the output volumetric flow Fo. Additionally, the control unit communicates with heat exchanger 2, more preferably with heating element 3 and/or cooling element 4 and controls these elements, for instance by providing the necessary electrical power input.

Fig. 2 shows a device comprising also heat exchanger 2, control unit 11, peristaltic pumps 8, 9 merging element 10 and fluid connections 12, 11 corresponding to the device shown in Fig. 1. Additionally, the device is in fluid communication not only with one fluid supply 1 but with two fluid supplies 1, which may be in communication with heat exchanger 2 so that one incoming flow is provided to heat exchanger 2. Alternatively, the fluid of the two fluid supplies 1 may be supplied directly to heating element 3 and cooling element 4, respectively. In this device, heat exchanger 2 may comprise a Peltier element 14 comprising a warm side and a cold side. The warm side represents heating element 3 and the cold side represents cooling element 4. Such Peltier element may also be foreseen in the embodiment according to Fig. 1.

The invention also covers all further features shown in the figures individually although they may not have been described in the afore description. The present invention covers further embodiments with any combination of features from different embodiments described above. Reference signs in the claims are provided merely as a clarifying example and shall not be construed as limiting the scope of the claims in any way. The present invention also covers the exact terms, features, values and ranges etc. in case these terms, features, values and ranges etc. are used in conjunction with terms such as about, around, generally, substantially, essentially, at least etc. (i.e., "about 3" shall also cover exactly 3 or "substantial constant" shall also cover exactly constant). The terms "a", "an", "first", "second" etc do not preclude a plurality.

**Claims**

1. Method for adjusting the temperature of medical liquids, comprising:

   Providing an incoming volumetric flow (Fi) from a fluid supply (1);
   Separating the incoming volumetric flow (Fi) into two partial volumetric flows (F1, F2);
   Adjusting the fluid temperature (T1, T2) of each of the partial volumetric flows (F1, F2) to substantially constant target temperatures (Tt1, Tt2) of the partial volumetric flows (F1, F2); and
   volumetric flow controlled merging of the partial volumetric flows (F1, F2) to an output volumetric flow (Fo).

2. The method of claim 1, wherein the fluid temperature (T1,T2) of the partial volumetric flows (F1, F2) is adjusted with a heat exchanger (2), the heat exchanger (2) being adapted to provide the substantially constant target temperatures (Tt1, Tt2) of the partial volumetric flows up to a respective maximum partial volumetric flow (Fm1, Fm2).

3. The method of any one of the preceding claims, wherein the heat exchanger (2) comprises a heating element (3) and a cooling element (4).

4. The method of any one of the preceding claims, wherein the fluid supply (1) and/or the partial volumetric flows (F1, F2) substantially has/have the same incoming temperature (Ti, Ti1, Ti2).

5. The method of any one of the preceding claims, wherein the electrical power input of the heat exchanger (2) is controlled based on the partial volumetric flows (F1, F2).

6. The method of any one of the preceding claims, wherein the heat exchanger (2) comprises a Peltier element.

7. The method of any one of the preceding claims, wherein the heat exchanger (2) comprises a buffer or two buffers (5, 6), the buffer(s) (5, 6) being associated with the partial volumetric flows (F1, F2) and adapted for heating and/or cooling the partial volumetric flows (F1, F2).

8. The method of claim 7, wherein the buffer(s) (5, 6) is/are adapted to the temperature variance within itself; and/or are adapted to offset or reduce the thermal inertia of the heat exchanger (2).

9. Device for adjusting the temperature of medical liquids, comprising:

a separator (7) being connectable to a fluid supply (1) separating an incoming volumetric flow (Fi) into two partial volumetric flows (F1, F2);
a heat exchanger (2) adapted to change the fluid temperature (T1, T2) of each of the partial volumetric flows (F1, F2) to substantially constant target temperatures (Tt1, Tt2) of the partial volumetric flows(F1, F2);
two volumetric flow actuators (8, 9) controlling the partial volumetric flows (F1, F2); and
an merging element (10) merging the partial volumetric flows (F1, F2) to an output volumetric flow (Fo).

10. The device of claim 9, wherein the heat exchanger (2) is adapted to provide the substantially constant target temperatures (Tt1, Tt2) of the partial volumetric flows up to the maximum partial volumetric flows (Fm1, Fm2).

11. The device of claims 9 or 10, wherein the heat exchanger (2) comprises a heating element (3) and a cooling element (4).

12. The device of any one of the preceding claims 9 to 11, wherein the electrical power input of the heat exchanger (2) is controlled based on the partial volumetric flows (F1, F2); and/or
wherein the heat exchanger (2) comprises a Peltier element.

13. The device of any one of the preceding claims 9 to 12, wherein the heat exchanger comprises a buffer or two buffers (5, 6) being associated with the partial volumetric flows (F1, F2) and adapted for heating and/or cooling the partial volumetric flows (F1, F2).

14. The device of claim 13, wherein the buffer(s) (5, 6) is/are adapted to the temperature variance within itself, and/or are adapted to offset or reduce the thermal inertia of the heat exchanger (2).

15. The device of any one of the preceding claims 9 to 14, wherein the two volumetric flow actuators are speed controlled pumps (8, 9), preferably peristaltic pumps (8, 9).

Fig. 1

Fig. 2

**EUROPEAN SEARCH REPORT**

Application Number

EP 12 18 0279

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2009/056640 A2 (BAENKLER MARC [DE]; ROTH MATTHIAS [DE]; KOLLMAR RAINER [DE]) 7 May 2009 (2009-05-07) * the whole document * | 1-15 | INV. A61M5/44 |
| A | US 2008/154197 A1 (DERRICO JOEL BRIAN [US] ET AL) 26 June 2008 (2008-06-26) * the whole document * | 1-15 | |
| A | US 2006/030917 A1 (ECCLESTON ROLANDO A [US] ET AL) 9 February 2006 (2006-02-09) * the whole document * | 1-15 | |
| A | WO 00/16722 A1 (ESTILL MEDICAL TECHNOLOGIES IN [US]) 30 March 2000 (2000-03-30) * the whole document * | 1-15 | |

TECHNICAL FIELDS
SEARCHED       (IPC)

A61M

The present search report has been drawn up for all claims

| Place of search | Date of completion of the search | Examiner |
|---|---|---|
| Munich | 21 March 2013 | Neiller, Frédéric |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 12 18 0279

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2013

| Patent document cited in search report | | Publication date | Patent family member(s) | | Publication date |
|---|---|---|---|---|---|
| WO 2009056640 | A2 | 07-05-2009 | EP | 2203136 A2 | 07-07-2010 |
| | | | WO | 2009056640 A2 | 07-05-2009 |
| US 2008154197 | A1 | 26-06-2008 | NONE | | |
| US 2006030917 | A1 | 09-02-2006 | CN | 1730108 A | 08-02-2006 |
| | | | EP | 1623733 A2 | 08-02-2006 |
| | | | JP | 2006043466 A | 16-02-2006 |
| | | | US | 2006030917 A1 | 09-02-2006 |
| WO 0016722 | A1 | 30-03-2000 | AU | 749480 B2 | 27-06-2002 |
| | | | AU | 6151299 A | 10-04-2000 |
| | | | BR | 9913822 A | 02-10-2001 |
| | | | CA | 2344416 A1 | 30-03-2000 |
| | | | CN | 1326334 A | 12-12-2001 |
| | | | EP | 1113770 A1 | 11-07-2001 |
| | | | IL | 141980 A | 15-05-2007 |
| | | | JP | 4312388 B2 | 12-08-2009 |
| | | | JP | 2002526198 A | 20-08-2002 |
| | | | MX | PA01002721 A | 04-06-2002 |
| | | | TW | 410163 B | 01-11-2000 |
| | | | US | 6142974 A | 07-11-2000 |
| | | | WO | 0016722 A1 | 30-03-2000 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009056640 A